(19) Europäisches Patentamt
European Patent Office
Office européen des brevets

(11) **EP 2 455 373 A1**

(12) **EUROPEAN PATENT APPLICATION**

(43) Date of publication:
**23.05.2012 Bulletin 2012/21**

(51) Int Cl.:
*C07D 307/42* *(2006.01)* *C10L 1/02* *(2006.01)*

(21) Application number: **12155899.3**

(22) Date of filing: **05.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **07.09.2007 EP 07075778**

(62) Document number(s) of the earlier application(s) in accordance with Art. 76 EPC:
**08785854.4 / 2 197 862**

(71) Applicant: **Furanix Technologies B.V**
**1014 BV Amsterdam (NL)**

(72) Inventor: **Gruter, Gerardus Johannes Maria**
**1014 BV Amsterdam (NL)**

(74) Representative: **Zeestraten, Albertus W. J.**
**Exter Polak & Charlouis B.V. (EP&C)**
**P.O. Box 3241**
**2280 GE Rijswijk (NL)**

Remarks:
This application was filed on 17-02-2012 as a divisional application to the application mentioned under INID code 62.

(54) **5-(substituted methyl) 2-methylfuran**

(57) The current invention provides a method for the manufacture of 5-(substituted methyl) 2-methylfuran by reacting 5-(substituted methyl) furfural with hydrogen and a catalyst system, comprising of one or more catalysts. Within the scope of the current invention is the use of 5-(substituted methyl) furfural, and in particular 5-hydroxymethylfurfural and the ethers or esters thereof, which may be obtained from C6 sugars.

EP 2 455 373 A1

**Description**

Technical Field

The present invention concerns a method for the manufacture of a 5-(substituted methyl) 2-(methyl)furan (or a mixture of such furans) from by reacting a starting material comprising at least a 5-(substituted methyl) furfural with hydrogen in the presence of a catalyst system. The invention also concerns a method for the manufacture of mixtures of 5-(substituted methyl) 2-(methyl)furan(s) and 2-methylfuran by reacting a starting material further comprising furfural. The invention also concerns the use of the products or product mixtures obtained by the method according to the invention as a fuel or a fuel additive.

Background Art

Fuel, fuel additives and various chemicals used in the petrochemical industry are derived from oil, gas and coal, all finite sources. Biomass, on the other hand, is considered a renewable source. Biomass is biological material (including biodegradable wastes) which can be used for the production of fuels or for industrial production of e.g. fibres, chemicals or heat. It excludes organic material which has been transformed by geological processes into substances such as coal or petroleum.

Production of biomass derived products for non-food applications is a growing industry. Biobased fuels are an example of an application with strong growing interest.

Biomass contains sugars (hexoses and pentoses) that may be converted into value added products. Current biofuel activities from sugars are mainly directed towards the fermentation of sucrose or glucose into ethanol or via complete breakdown via Syngas to synthetic liquid fuels. EP 0641 854 describes the use of fuel compositions comprising of hydrocarbons and/or vegetable oil derivatives containing at least one glycerol ether to reduce particulate matter emissions.

More recently, the acid catalysed reaction of fructose has been re-visited, creating HMF as an intermediate of great interest. Most processes investigated have the disadvantage that HMF is not very stable at the reaction conditions required for its formation. Fast removal from the water-phase containing the sugar starting material and the acid catalyst has been viewed as a solution for this problem. Researchers at the University of Wsconsin-Madison have developed a process to make HMF from fructose. HMF can be converted into monomers for plastics, petroleum or fuel extenders, or even into fuel itself. The process by prof. James Dumesic and co-workers first dehydrates the fructose in an aqueous phase with the use of an acid catalyst (hydrochloric acid or an acidic ion-exchange resin). Salt is added to salt-out the HMF into the extracting phase. The extracting phase uses an inert organic solvent that favors extraction of HMF from the aqueous phase. The two-phase process operates at high fructose concentrations (10 to 50 wt %), achieves high yields (80% HMF selectivity at 90% fructose conversion), and delivers HMF in a separation-friendly solvent ( DUMESIC, James A, et al. "Phase modifiers promote efficient production of Hydroxymethylfurfural from fructose" . Science. 30 juni 2006, vol.312, no.5782, p.1933-1937). Although the HMF yields from this process are interesting, the multi-solvent process has cost-disadvantages due to the relatively complex plant design and because of the less than ideal yields when cheaper and less reactive hexoses than fructose, such as glucose or sucrose, are used as a starting material. HMF is a solid at room temperature which has to be converted in subsequent steps to useful products. Dumesic has reported an integrated hydrogenolysis process step to convert HMF into dimethylfuran (DMF), which is assumed to be an interesting gasoline additive.

In WO 2006/063220 a method is provided for converting fructose into 5- ethoxymethylfurfural (EMF) at 60 °C, using an acid catalyst either in batch during 24 hours or continuously via column elution during 17 hours. Applications of EMF were not discussed.

Also in copending patent application PCT/EP2007/002145 the manufacture of HMF ethers are described, including the use of such ethers as fuel or fuel additive. Indeed, both the methyl ether and the ethyl ether (methoxymethylfurfural, or MMF; ethoxyethylfurfural or EMF) were prepared and tested. A similar case is co-pending patent application PCT/EP2007/002146, which describes the manufacture of HMF esters, such as acetylmethylfurfural (AMF).

Moreover, it is known to make furfural from the polysaccharide hemicellulose, a polymer of sugars containing five carbon atoms each. When heated with sulphuric acid, hemicellulose undergoes hydrolysis to yield these sugars, principally xylose. Under the same conditions of heat and acid, xylose and other five carbon sugars undergo dehydration, losing three water molecules to become furfural:

$$C_5H_{10}O_5 \rightarrow C_5H_4O_2 + 3\ H_2O$$

Although MMF, EMF, AMF and other ethers and esters of HMF and furfural are useful as fuel or fuel additives, the inventors found that these ethers and esters leave room for improvement, in particular when used in higher concentration blends with fuels such as gasoline, kerosene, diesel, biodiesel or green diesel. The inventors have therefore set out to overcome this shortfall. It is known that HMF may be converted into 2,5-dimethylfuran. For instance, in "Production of dimethylfuran for liquid fuels from biomass-derived carbohydrates", Nature, vol. 447 (21 June 2007), pp. 982-985, James Dumesic et al. describes the conversion of fructose into HMF, which is subsequently converted into several hydrogenation steps via 2,5-dihydroxymethylfuran and 2-methyl-5-hydroxymethylfuran into DMF. Thus, a large amount of hydrogen is required to generate a liquid fuel suitable for the transportation sector.

Surprisingly, the current inventors found that the conversion of HMF into DMF is not required in order to prepare a product with a high energy density, suitable boiling point and suitable solubility. Moreover, suitable fuel or fuel additives may even be made from furfural, HMF ethers such as EMF and HMF esters such as AMF and/or mixtures containing these components with smaller amounts of hydrogen and without losing molecular mass but with adding molecular mass to the products. This would therefore provide a route to an alternative fuel or fuel additive from a renewable (and hence CO2 neutral) source.

Disclosure of Invention

Accordingly, the current invention provides a method for the manufacture of 5-substituted 2-methylfuran by reacting 5-(substituted methyl) furfural with hydrogen and a catalyst system, comprising of one or more catalysts. Within the scope of the current invention is the use of 5-(substituted methyl) furfural, and in particular 5-hydroxymethylfurfural and the ethers or esters thereof, which may be obtained from C6 sugars. The synthesis of furfural (from C5 sugars) and/or of the substituted furfural is not part of the current invention. It is noted, however, that the current process is ideally suitable for the manufacture of fuel components or additives from feed containing ethers of 5-(substituted methyl) furfural and optionally unsubstituted furfural, which in turn could be obtained from a mixed pentose and hexose containing biomass source.

When the reaction product of the above method is used as such or when it is used as an intermediate for a subsequent conversion, the selectivity of the reaction is preferably high as the product is preferably pure. However, when the reaction product of the above method is used as a fuel, a fuel additive or as a fuel or a fuel additive intermediate, the reaction product does not necessarily need to be pure. Indeed, in the preparation of fuel and fuel additives from biomass, which in itself is a mixture of various pentoses and hexoses is an advantage. Next to the 5-substituted 2-methylfuran and 2-methylfuran, the reaction product may contain additional non-interfering components such as levulinic acid derivatives and/or products of non-selective hydrogenation such as dimethylfuran and tetrahydrofurans and the like. For ease of reference, however, the method and the reaction product of the current invention are described in terms of the reaction of a 5-substituted furfural starting material to the 5-substituted 2-methylfuran. The current invention also provides for the use of the reaction product made according to the present invention as fuel or as fuel additive. Fuels for blending with the product of the present invention include but are not limited to gasoline and gasoline-ethanol blends, kerosene, diesel, biodiesel (refers to a non-petroleum-based diesel fuel consisting of short chain alkyl (methyl or ethyl) esters, made by transesterification of vegetable oil, which can be used (alone, or blended with conventional petrodiesel), Fischer-Tropsch liquids (for example obtained from GTL, CTL or BTL gas-to-liquids/coal-to-liquids/biomass to liquids processes), diesel-biodiesel blends and green diesel and blends of diesel and/or biodiesel with green diesel (green diesel is a hydrocarbon obtained by hydrotreating biomass derived oils, fats, greases or pyrolysis oil; see for example the UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085). The product is a premium diesel fuel containing no sulfur and having a cetane number of 90 to 100). Fuels for blending with the product of the present invention may also include one or more other furanics, wherein the expression furanics is used to include all derivatives of furan and tetrahydrofuran. The invention also provides a fuel composition comprising a fuel element as described above and the reaction product made according to the present invention.

Mode(s) for Carrying Out the Invention

The synthesis of HMF from fructose, glucose and sucrose as a biomass source is a hot topic. HMF has been obtained in processes using both homogeneous and heterogeneous catalysts, using different diluent systems such as water, 2 phase systems for extracting the HMF into an organic phase after its formation, or using diluent systems such as acetone, dmso or ionic liquids.

The current method provides for the conversion of 5-substituted furfural into 5-substituted 2-methylfuran and as furfural may be present when pentoses were present in the sugar dehydration step or when furfural is formed during hexose dehydration, the current method also provides for the concurrent conversion of the furfural into methylfuran.

The catalyst system used in the method of the present invention may comprise one or more (co)catalysts, and preferably is a hydrogenation catalyst. The hydrogenation catalyst is preferably a heterogeneous catalyst (meaning a solid catalyst). Suitably, it is a granular catalyst which may be formed into any suitable shape, e.g. pellets, rings or saddles. Hydrogenation catalysts for aldehydes are known and believed suitable in the method of the current invention. Typical aldehyde hydrogenation catalysts include copper-containing catalysts and Group VIII metal-containing catalysts. Examples of suitable copper-containing catalysts include copper-on-alumina catalysts, reduced copper oxide/zinc oxide catalysts, with or without a promoter, manganese promoted copper catalysts, and reduced copper chromite catalysts, with or without a promoter, while suitable Group VIII metal-containing catalysts include platinum, rhodium, ruthenium and palladium catalysts, preferably on a refractory support such as carbon, silica, alumina, aluminasilica, a carbonate such as barium carbonate, diatomaceous earth and the like.

Suitable copper oxide/zinc oxide catalyst precursors include CuO/ZnO mixtures wherein the Cu:Zn weight ratio ranges from about 0.4:1 to about 2:1. Promoted copper oxide/zinc oxide precursors include CuO/ZnO mixtures wherein the Cu:Zn weight ratio ranges from about 0.4:1 to about 2:1 which are promoted with from about 0.1 % by weight up to about 15% by weight of barium, manganese or a mixture of barium and manganese. Suitable copper chromite catalyst precursors include those wherein the Cu:Cr weight ratio ranges from about 0.1:1 to about 4:1, preferably from about 0.5:1 to about 4:1. Promoted copper chromite precursors include copper chromite precursors wherein the Cu:Cr weight ratio ranges from about 0.1:1 to about 4:1, preferably from about 0.5:1 to about 4:1, which are promoted with from about 0.1 % by weight up to about 15% by weight of barium, manganese or a mixture of barium and manganese. Manganese promoted copper catalyst precursors typically have a Cu:Mn weight ratio of from about 2:1 to about 10:1 and can include an alumina support, in which case the Cu:Al weight ratio is typically from about 2:1 to about 4:1.

Other catalysts which can be considered for use include Pd/ZnO catalysts of the type mentioned by P. S. Wehner and B. L. Gustafson in Journal of Catalysis 136, 420-426 (1992), supported palladium/zinc catalysts of the type disclosed in U.S. Pat. No. 4,837,368 and U.S. Pat. No. 5,185,476, and chemically mixed copper-titanium oxides of the type disclosed in U.S. Pat. No. 4,929,777.

Further catalysts of interest for use in the process of the invention include the rhodium/tin catalysts reported in A. El Mansour, J. P. Candy, J. P. Bournonville, O. A. Ferrehi, and J. M Basset, Angew. Chem. 101, 360 (1989).

Any recognised supporting medium may be used to provide physical support for the catalyst used in the process of the invention. This support can be provided by materials such as zinc oxide, alumina, silica, aluminasilica, silicon carbide, zirconia, titania, carbon, a zeolite, or any suitable combination thereof. Particularly preferred are catalyst systems comprising a Group VIII metal ("noble metal") on a carbon support.

The amount of catalyst may vary, depending on the selection of catalyst or catalyst mixture. For instance, the catalyst can be added to the reaction mixture in an amount varying from 0.01 to 40 mole % drawn on the (substituted) furfural content of the starting material, preferably from 0.1 to 30 mole %, more preferably from 1 to 20 mole %.

In the preferred embodiment, the catalyst is a heterogeneous catalyst.

The temperature at which the reaction is performed may vary, but in general it is preferred that the reaction is carried out at a temperature from 0 to 200 degrees Celsius, preferably from 10 to 150 degrees Celsius, more preferably from 20 to 120 degrees Celsius. Also, the hydrogenation reaction is most selective at low temperatures such as e.g. between 20 and 80 degrees Celsius, depending on the selected catalyst.

Hydrogen is supplied is sufficient abundance, and either bubbled through the reaction medium introduced concurrently or counter currently with one of the feed streams or dissolved using another form of mixing. Depending on the catalyst and the selected process temperature, the reaction is carried out at a hydrogen pressure from 1 to 100 bars, preferably from 2 to 25 bars, more preferably from 2 to 10 bars. In general, pressures higher than 100 bars are less preferred as the selectivity of the reaction reduces and too much hydrogen is consumed for by-products formation.

The furfural, HMF and HMF ether and ester containing starting material is typically dissolved or suspended in a non-reactive solvent which may be selected form the group consisting of organic solvents such as, ketones, ethers, alcohols, alkanes and the like. When the solvent is an alcohol, the hydrogenation catalyst should not catalyze etherification reactions.

The method of the current invention may be carried out in a batch process or in a continuous process, with or without recycle of (part of) the product stream to control the reaction temperature (recycle via a heat exchanger). For instance, the method of the invention can be performed in a continuous flow process. In such method, one or two homogenous catalysts may be used and the residence time of the reactants in the flow process is between 0.1 second and 10 hours, preferably from 1 second to 1 hours, more preferably from 5 seconds to 20 minutes.

Alternatively, the continuous flow process may be a fixed bed continuous flow process or a reactive (catalytic) distillation process with a heterogeneous acid catalyst. To initiate or regenerate the heterogeneous acid catalyst or to improve performance, an inorganic or organic acid may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100 min$^{-1}$.

The above process results in stable 5-substituted methylfurans, which can then be used as such or be converted into a further derivative before being used as fuel and/or as fuel additive. The inventors are of the opinion that some of the products prepared by the method of the current invention are actually new. Thus, the 5-substituted methylfurfural with as substituent on the 5-position a C4 to C10,alkoxymethyl substituent are new and are excellent fuel components or fuel additives. Since these products are made from biomass, this might open a class of products that are fully biomass-derived. Accordingly, these new 5-substituted 2-methylfurans are claimed as well.

The 5-substituted 2-methylfurans of the invention can also be used as or can be converted to compounds that can be used as solvent, as monomer in a polymerization (such as 2,5-furan dicarboxylic acid or FDCA), as fine chemical or pharmaceutical intermediate, or in other applications. Oxidation of the 5-substituted 2-methylfurans using an appropriate catalyst under appropriate conditions such as for example described for p-xylene with a NHPI/Co(OAC)$_2$/MnOAC$_2$ catalyst system in Adv. Synth. Catal. 2001, 343, 220-225 or such as described for HMF with a Pt/C catalyst system at pH < 8 in EP 0 356 703 or or such as described for HMF with a Pt/C catalyst system at pH > 7 in FR 2 669 634, all with air as an oxidant, resulted in the formation of 2,5-furan dicarboxylic acid (FDCA).

The invention further concerns the use of the 5-substituted 2-methylfurans prepared by the method of the current invention as fuel and/or as fuel additive. Of particular interest is the use of the 5-substituted 2-methylfurans in diesel, biodiesel or "green diesel", given its (much) greater solubility therein than ethanol. Conventional additives and blending agents for diesel fuel may be present in the fuel compositions of this invention in addition to the above mentioned fuel components. For example, the fuels of this invention may contain conventional quantities of conventional additives such as cetane improvers, friction modifiers, detergents, antioxidants and heat stabilizers, for example. Especially preferred diesel fuel formulations of the invention comprise diesel fuel hydrocarbons and 5-substituted 2-methylfurans as above described together with peroxidic or nitrate cetane improvers such as ditertiary butyl peroxide, amyl nitrate and ethyl hexyl nitrate for example.

Examples are enclosed to illustrate the method of the current invention and the suitability of the products prepared therefrom as fuel. The examples are not meant to limit the scope of the invention.

**Example 1. Formation of 5-(ethoxymethyl)-2-methylfuran**

In a 7.5 ml batch reactor, 0.06 mmol 5-(ethoxymethyl)furfural (EMF) in ethanol/H$_2$O (90/10) or diglyme/H$_2$O (90/10) and 3.3 mmol H$_2$ was reacted for 1, 2 or 18 hours at a temperature of 150 or 80 degrees Celsius with 5 mg heterogeneous hydrogenation catalyst and in some cases 5 mg acid catalyst. Four furan peaks were observed in the UV spectrum. Mass spectrometry (LC-MS CI) identified these products as 5-(ethoxymethyl)furfural (EMF; starting material), 2,5-di(ethoxymethyl)furan (DEMF), 5-(ethoxymethyl)-2-(hydroxymethyl)furan (EMHMF) and 5-(ethoxymethyl)-2-methylfuran (EMMeF).

Conversion of substrate, selectivity and yield of furan derivatives were calculated according to the following formulae:

$$X=100*m_{r\ substrate}/m_{0\ substrate}$$

| | |
|---|---|
| X | conversion (%) |
| $m_{r\ substrate}$ | amount of reacted substrate (mg) |
| $m_{0\ substrate}$ | amount of substrate in feed (mg) |

$$S_{compound}=100*n_{r\ substrate}/n_{0\ substrate}$$

| | |
|---|---|
| $S_{compound}$ | selectivity to compound (%) |
| $n_{r\ substrate}$ | moles of substrate reacted |
| $n_{0\ substrate}$ | moles of substrate in feed |

$$Yield=100*n_{product}/n_{0\ substrate}$$

| | |
|---|---|
| Yield | yield (%) |
| $n_{product}$ | moles of product formed |

Selectivities and conversions for catalysts used in this example can be found in table below.

Table 1. Conversions and selectivities for the hydrogenolysis of 5-(ethoxymethyl)furfural in different solvents, temperatures and reaction times.

| Catalyst 1 | Catalyst 2 | Solvent | T [°C] | Reaction Time [h] | Conversion [%] | sEMHMF [%] | sEMMeF[ %] | s Further hydrogenated products [%] |
|---|---|---|---|---|---|---|---|---|
| Ni/MoO3 on silica-alumina | None | Diglyme | 150 | 18 | 46.2 | 1.8 | 10.7 | 87.5 |
| 5%Pt on active C | None | Diglyme | 150 | 18 | 99.5 | 0.1 | 10.7 | 89.2 |
| 5% Ru on active C | None | EtOH | 150 | 2 | 92.7 | 0.5 | 11.9 | 87.7 |
| 5%Pt/0.5%V on C | Amberlyst36Wet | EtOH | 80 | 1 | 99.8 | 6.5 | 10.7 | 82.7 |
| 1.85%Ru on silica | Bentonite | EtOH | 80 | 1 | 14.3 | 86.9 | 12.2 | 0.9 |

**Analytical Method**

The reaction products were quantified with the aid of HPLC-analysis with an internal standard (saccharine, Sigma Aldrich). An Agilent 1100 series chromatograph, equipped with UV and ELSD detectors, was used. Stationary phase was reverse phase C18 (Sunfire 3.5 μm, 4.6x100mm, Waters) column. A gradient elution at a constant flow 0.6 ml/min and temperature 40 °C was used according to the following scheme.

| Time | H2O(vol%) | MeOH (vol%) | MeCN (vol%) | Flow (ml/min) | T (C) |
|---|---|---|---|---|---|
| Initial | 95 | 0 | 5 | 1 | 40 |
| 1 | 89 | 3 | 8 | 1 | 40 |
| 8 | 25 | 3 | 72 | 1 | 40 |

Mass spectrum of EMHMF (MW=156.2)- only peaks of $H_2O$ subtract and adduct are visible (MW=139 and 175 respectively). Mass spectrum of EMMeF (MW=139.2).

**Example 2.**

Hydrogenation of aldehyde

A teflon lined, 7.5 mL stainless steel batch reactor containing 150 mg (1.0 mmol) of 5-(ethoxymethyl)furfural in 0.64 mL dioxane and 10.4 mg of a CuCr catalyst is pressurized to 50 bar of hydrogen and subsequently heated, under stirring, to 150 °C for 3 hours. After the reaction, de reactor is cooled quickly in an ice bath and depressurized. A sample is diluted with methanol for analysis of the products with GC and GC-MS. The analysis shows a 2-(ethoxymethyl)furfural conversion of 100 %, a selectivity to 2-(ethoxymethyl)-5-methylfuran of 18.7%, a selectivity to 2-(ethoxymethyl)-5-(hydroxymethyl)furan of 62%, and a selectivity to ring hydrogenated products (mainly 2-(ethoxymethyl)-5-methyltetrahydrofuran) of 1.0 %. In this experiment, the selectivity was calculated slightly different, based on the formula:

$$\text{Selectivity} = 100 * n_t(\text{product})/[n_0(\text{substrate} - n_t(\text{substrate})]$$

Where:

$n_0$ - the initial number of moles
$n_t$ - the number of moles of a compound at time "t".

**Example 3. Diesel fuel applications**

Fuel solubility

Fuel solubility is a primary concern for diesel fuel applications. Not all highly polar oxygenates have good solubility in the current commercial diesel fuels. Results show that 5-(ethoxymethyl)-2-methylfuran and 5-(tertbutoxymethyl)-2-methylfuran are miscible in all blend ratio's with commercial diesel. In a comparative set of experiments it was shown that ethoxymethylfurfural (EMF) is completely miscible in a 5 vol% blend with commercial diesel, but that phase separation occurs with the 25 vol% and with the 40 vol% blends of EMF and diesel.

References

- DUMESIC, James A, et al. "Phase modifiers promote efficient production of Hydroxymethylfurfural from fructose" . Science. 30 June 2006, vol.312, no.5782, p.1933-1937.
- WO 2006/063220
- Chapter 15 of Advanced Organic Chemistry, by Jerry March, and in particular under reaction 5-4. (3rd ed., © 1985 by John Wiley & Sons, pp. 684-685).
- MOREAU, Claude, et al. "Dehydration of fructose and sucrose into 5-hydroxymethylfurfural in the presence of 1-H-3-methyl imidazolium chloride acting both as solvent and catalyst", Journal of Molecular Catalysis A: Chemical 253 (2006) p. 165-169.

- EP 0641 854
- UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085))
- Adv. Synth. Catal. 2001, 343, 220-225
- EP 0 356 703
- FR 2 669 634
- P. S. Wehner and B. L. Gustafson in Journal of Catalysis 136, 420-426 (1992)
- U.S. Pat. No. 4,837,368
- U.S. Pat. No. 5,185,476
- U.S. Pat. No. 4,929,777.
- A. El Mansour, J. P. Candy, J. P. Bournonville, O. A. Ferrehi, and J. M Basset, Angew. Chem. 101, 360 (1989).

**Claims**

1. Method for the manufacture of a 5-(substituted methyl) 2-methylfuran by reacting a starting material comprising at least one 5-(substituted methyl) furfural and optionally comprising furfural with hydrogen in the presence of a catalyst system.

2. Method according to claim 1, wherein the catalyst system is a heterogeneous hydrogenation catalyst

3. Method according to claim 3, wherein the heterogeneous hydrogenation catalyst contains at least one noble metal on a carbon support.

4. Method according to any one of the claims 1 to 3, wherein the reaction is performed at a temperature from 0 to 200 degrees Celsius, preferably from 10 to 150 degrees Celsius, more preferably from 20 to 120 degrees Celsius.

5. Method according to any one of the claims 1 to 4, wherein the starting material is selected from one or more of the group comprising ethers and esters of 5-(hydroxymethyl)furfural, optionally comprising 5-hydroxymethyl furfural and/or furfural.

6. Method according to any one of the claims 1 to 5, performed in the presence of a solvent, wherein a solvent is used, and wherein the solvent is selected from the group consisting of ketones, ethers, alkanes and aromatic hydrocarbons and mixtures thereof.

7. Method according to any one of the claims 1 to 6, wherein the method is performed in a continuous flow process.

8. Method according to claim 7, wherein the residence time in the flow process is between 0.1 second and 10 hours, preferably from 1 second to 1 hours, more preferably from 5 seconds to 20 minutes.

9. Method according to claim 8, wherein the continuous flow process is a fixed bed continuous flow process.

10. Method according to claim 9, wherein the fixed bed comprises a heterogeneous acid catalyst.

11. Method according to claim 10, wherein the continuous flow process is a reactive distillation or a catalytic distillation process.

12. Method according to claim 11, wherein in addition to a heterogeneous acid catalyst, an inorganic or organic acid catalyst is added to the feed of the fixed bed or catalytic distillation continuous flow process.

13. Method according to claim 9-12, wherein the liquid hourly space velocity ("LHSV") is from 1 to 1000, preferably from 5 to 500, more preferably from 10 to 250 and most preferably from 25 to 100 $min^{-1}$.

Europäisches
Patentamt

European
Patent Office

Office européen
des brevets

**EUROPEAN SEARCH REPORT**

Application Number

EP 12 15 5899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | D. DINELLI, G. B. MARINI-BETTOLO: GAZZETTA CHIMICA ITALIANA, vol. LXXI, 1941, pages 117-128, XP009095946, * page 127 * | 1-13 | INV. C07D307/42 C10L1/02 |
| X,D | Y. ROMAN-LESHKOV ET AL: NATURE, vol. 447, 21 June 2007 (2007-06-21), pages 982-985, XP002469198, * the whole document * | 1-3,7-9, 13 | |
| Y | R. S. RAO: CATALYSIS LETTERS, vol. 60, 1999, pages 51-57, XP002469199, * the whole document * | 1-13 | |
| Y | ZHENG ET AL: "Towards understanding the reaction pathway in vapour phase hydrogenation of furfural to 2-methylfuran", JOURNAL OF MOLECULAR CATALYSIS. A, CHEMICAL, ELSEVIER, AMSTERDAM, NL, vol. 246, no. 1-2, 1 March 2006 (2006-03-01), pages 18-23, XP005299075, ISSN: 1381-1169 * the whole document * | 1-13 | TECHNICAL FIELDS SEARCHED (IPC) C07D C10L |
| Y | D. G. MANLY, A. P. DUNLOP: JOURNAL OF ORGANIC CHEMISTRY, vol. 23, 1958, pages 1093-1095, XP002469208, * the whole document * | 1-13 | |
| Y | WO 02/34697 A (PURE ENERGY CORP [US]) 2 May 2002 (2002-05-02) * the whole document * | 1-13 | |
| | -/-- | | |

The present search report has been drawn up for all claims

| Place of search | Date of completion of the search | Examiner |
|---|---|---|
| Munich | 22 March 2012 | Nikolai, Joachim |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 12 15 5899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YOSHIDA K ET AL: "Concurrent Anodic Cyanation and Methoxylation of Methylated Furans. Oxidation Potential and Reactivity, and Stereochemical Control of Addition", BULLETIN OF THE CHEMICAL SOCIETY OF JAPAN, CHEMICAL SOCIETY OF JAPAN, TOKYO, JP, vol. 60, no. 1, 1 January 1987 (1987-01-01), pages 229-240, XP002513867, ISSN: 0009-2673, DOI: 10.1246/BCSJ.60.229 * compound 2M * | 1-13 | |
| A | EP 0 082 689 A (BRITISH PETROLEUM CO PLC [GB]) 29 June 1983 (1983-06-29) * claim 4 * * example 2 * | 1-13 | |
| A | DAVIES HUW M L ET AL: "Mechanistic Aspects of Formal ü3+4] Cycloadditions between Vinylcarbenoids and Furans", TETRAHEDRON, ELSEVIER SCIENCE PUBLISHERS, AMSTERDAM, NL, vol. 43, no. 19, 1 January 1987 (1987-01-01), pages 4265-4270, XP002513868, ISSN: 0040-4020, DOI: 10.1016/S0040-4020(01)90301-1 * the whole document * | 1-13 | |
| A | FR 2 679 918 A (COSMO SOGO KENKYUSHO KK [JP]; COSMO OIL CO LTD [JP]) 5 February 1993 (1993-02-05) * claims 5,12 * | 1-13 | TECHNICAL FIELDS SEARCHED (IPC) |

The present search report has been drawn up for all claims

| Place of search | Date of completion of the search | Examiner |
|---|---|---|
| Munich | 22 March 2012 | Nikolai, Joachim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 12 15 5899

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2012

| Patent document cited in search report | | Publication date | Patent family member(s) | | Publication date |
|---|---|---|---|---|---|
| WO 0234697 | A | 02-05-2002 | AU | 2582602 A | 06-05-2002 |
| | | | US | 6479677 B1 | 12-11-2002 |
| | | | US | 2003018205 A1 | 23-01-2003 |
| | | | WO | 0234697 A2 | 02-05-2002 |
| EP 0082689 | A | 29-06-1983 | NONE | | |
| FR 2679918 | A | 05-02-1993 | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0641854 A
- WO 2006063220 A
- EP 2007002145 W
- EP 2007002146 W
- US 4837368 A

- US 5185476 A
- US 4929777 A
- EP 0356703 A
- FR 2669634

**Non-patent literature cited in the description**

- **DUMESIC, JAMES A et al.** Phase modifiers promote efficient production of Hydroxymethylfurfural from fructose. *Science,* 30 June 2006, vol. 312 (5782), 1933-1937
- **JAMES DUMESIC.** Production of dimethylfuran for liquid fuels from biomass-derived carbohydrates. *Nature,* 21 June 2007, vol. 447, 982-985
- **P. S. WEHNER ; B. L. GUSTAFSON.** *Journal of Catalysis,* 1992, vol. 136, 420-426
- **A. EL MANSOUR ; J. P. CANDY ; J. P. BOURNONVILLE ; O. A. FERREHI ; J. M BASSET.** *Angew. Chem.,* 1989, vol. 101, 360

- *Adv. Synth. Catal.,* 2001, vol. 343, 220-225
- **JERRY MARCH.** Advanced Organic Chemistry. John Wiley & Sons, 1985, 684-685
- **MOREAU, CLAUDE et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169